# EUROPÄISCHE PATENTSCHRIFT

(11) **EP 1 643 924 B1**
(45) Veröffentlichungstag und Bekanntmachung des Hinweises auf die Patenterteilung: **22.06.2011**
(21) Anmeldenummer: 04762360.8
(22) Anmeldetag: 09.07.2004
(51) Int. Cl.: A61B 18/20, A61F 9/008

(54) **Laser zur Bestrahlung biologischen Gewebes**
Laser for the irradiation of biological tissue
Laser pour l'irradiation de tissus biologiques

(30) Priorität: 11.07.2003 DE 10331792
(43) Veröffentlichungstag der Anmeldung: 12.04.2006
(73) Patentinhaber: Medizinisches Laserzentrum Lübeck GmbH, D-23562 Lübeck (DE)
(72) Erfinder: BRINKMANN, Ralph, 23562 Lübeck (DE); SCHUELE, Georg, Menlo Park, CA 94025 (US)
(74) Vertreter: Biehl, Christian
(86) Internationale Anmeldenummer: PCT/DE2004/001498
(87) Internationale Veröffentlichungsnummer: WO 2005/007002

(56) Entgegenhaltungen:
- WO-A-01/91661
- DE-A- 3 935 528
- DE-A- 10 135 944
- DE-A- 19 932 477
- SCHUELLE G ET AL: "OPTOACOUSTIC MEASUREMENTS DURING MUS-IRRADIATION OF THE RETINAL PIGMENT EPITHELIUM" PROCEEDINGS OF THE SPIE, SPIE, BELLINGHAM, VA, US, Bd. 3914, 22. Januar 2000 (2000-01-22), Seiten 230-236, XP001105005 ISSN: 0277-786X
- SCHUELE G ET AL: "OPTOACOUSTIC CONTROL SYSTEM FOR SELECTIVE TREATMENT OF THE RETINAL PIGMENT EPITHELIUM" PROCEEDINGS OF THE SPIE, SPIE, BELLINGHAM, VA, US, Bd. 4256, 23. Januar 2001 (2001-01-23), Seiten 71-76, XP001105006 ISSN: 0277-786X
- SCHUELE G ET AL: "OPTOACOUSTIC DETECTION OF SELECTIVE RPE CELL DAMAGE DURING MUS-LASER IRRADIATION" PROCEEDINGS OF THE SPIE, SPIE, BELLINGHAM, VA, US, Bd. 4433, 18. Juni 2001 (2001-06-18), Seiten 92-96, XP001105007 ISSN: 0277-786X

## Beschreibung

Die Erfindung betrifft einen Laser zur Behandlung biologischen Gewebes insbesondere einen Laser mit Dosimetriesteuerung nach dem Oberbegriff des Hauptanspruches, zum Beispiel zum Einsatz in der Augenmedizin, aber auch allgemein in der Therapie biologischen Gewebes.

In der Ophthalmologie finden Lasersysteme breite Anwendung sowohl bei der Korrektur der Sehschärfe (z.B. Hornhaut-Ablation, Lasik) als auch bei der Therapie von Netzhauterkrankungen. Hierzu sind Laser aufgrund der Transparenz des Auges für sichtbares Licht prädestiniert.

Besonderes Interesse besteht dabei an der Behandlung der Fehlfunktion des "Retinalen Pigmentepithels (RPE)", einer stark pigmentierten monozellulären Schicht zwischen Aderhaut und Netzhaut des Auges. Das hoch multifunktionale RPE regelt u.a. den Metabolismus der Photorezeptoren, den Flüssigkeitshaushalt der Netzhaut sowie die Phagozytose von Rhodopsin-Scheibchen, den Abbauprodukten des Sehvorgangs. Mit zunehmendem Alter werden metabolische Endprodukte der ständigen Rezeptorregeneration im RPE akkumuliert (u.a. Lipofuszine), was zu einer kontinuierlich abnehmenden Funktion dieser Zellschicht führt.

Beispielsweise ist die altersabhängige Makuladegeneration (AMD) mit steigender Inzidenz die häufigste Erblindungsursache von Menschen über 50 Jahren in den Industrienationen. Nach der Framingham-Eye-Study sind im Alter zwischen 52 und 64 Jahren 2%, zwischen 65 und 74 Jahren 11 % und über ein Drittel aller Menschen zwischen 75 und 84 Jahren betroffen, allein in den USA sind 4 bis 10 Millionen Patienten für das Jahr 2000 geschätzt. Die zweithäufigste Erblindungsursache ist die diabetische Retinopathie (DR), die als Spätfolge der Volkskrankheit Diabetes mellitus auftritt. Mit zunehmender Dauer der Erkrankung nimmt die Häufigkeit der Netzhautveränderungen zu und steigt nach 30 Jahren auf nahezu 100 %. Aufgrund des zunehmenden Alters der Menschen gewinnt eine möglichst frühzeitige, effiziente und schonende Therapie der AMD und DR zunehmend an Bedeutung.

An der Entstehung von AMD oder DR scheint das retinale Pigmentepithel zumindest beteiligt zu sein, da hier Gefäßneubildungen und Ödeme in der Umgebung des RPE entstehen, die bei funktionell intaktem RPE nicht auftreten sollten.

Die Laserbehandlung des dysfunktionalen RPE gerade auch im frühen Stadium einer Erkrankung ist grundsätzlich nicht unproblematisch. Zwar können durch Bestrahlung gezielt erkrankte Bereiche des Augenhintergrunds thermisch verödet werden, wobei durch anschließende Neubildung und laterale Proliferation von RPE-Zellen in die verödeten Zonen gute Aussichten auf eine weitgehende Wiederherstellung des intakten RPE besteht. Doch die Erzeugung der für die erkrankten Zellen tödlichen Temperaturen durch Laserlichtabsorption zieht für das umgebende Gewebe potentiell weitere Schädigungen nach sich, was insbesondere zum Absterben der nicht regenerierenden Photorezeptoren und damit zum permanenten Visusverlust führen kann.

Die selektive RPE-Therapie (SRT), die per Definition die Schädigung der RPE-Umgebung vermeidet, befindet sich bereits heute in einem fortgeschrittenen Entwicklungsstadium und hat gute Aussichten, sich in naher Zukunft als weit verbreitete Therapiemethode zu etablieren. Dies setzt allerdings voraus, daß ein optimiertes und leicht handhabbares Lasersystem am Markt bereitgestellt wird, wozu die vorliegende Erfindung beitragen soll.

Nach dem Vorschlag aus der DE 39 36 716 C2 empfiehlt sich zur selektiven Beeinflussung eines gut absorbierenden Materials in einer Matrix mit geringerem Absorptionsvermögen die repetierende Bestrahlung, also etwa die Anwendung gepulster Laserstrahlung. Das kurzzeitig aufeinander folgende Eintreffen einer Mehrzahl von Lichtpulsen lässt eine Erwärmung der Zielsubstanz zu, die ansonsten auch mit einem Einzelpuls deutlich höherer Energie zu erreichen wäre. Das zeitliche Auseinanderziehen der Energiedeposition erlaubt aber zugleich, Wärme- und Energietransportmechanismen im Material auszunutzen und den vom Licht kritisch erwärmten Bereich eng auf die Umgebung der Zielstrukturen zu begrenzen.

In der Anwendung dieses Konzepts auf die SRT wird zur Zeit ein Burst, d. i. eine Pulsfolge oder Pulssequenz, von ca. 30 Laserpulsen einer Pulsdauer von je 1.7 µs im grünen Spektralbereich mit einer Pulsfolgerate von 100 Hz bei einer Wellenlänge von 527 nm verwendet. Natürlich sind zahlreiche Variationen dieser Behandlungsparameter ebenfalls anwendbar. Für die Thermotherapie biologischen Gewebes, insbesondere des Augenhintergrunds, sind dabei aber Pulsdauern im Bereich weniger Mikrosekunden klar zu bevorzugen. Die Erzeugung solcher Laserpulse mit näherungsweise konstanter Pulsleistung ist in der DE 44 01 917 C2 dargelegt. Durch die starke Pigmentierung des RPE (etwa 50% des einfallenden Lichts im grünen Spektralbereich wird von den Pigmentgranula (Melanosomen) in den Zellen des RPE absorbiert) kommt es bei entsprechender Bestrahlung (pro Puls ca. 600 mJ/cm²) zu hohen Temperaturspitzen im RPE, die zu intrazellulärer Mikrovapoarisation an den stark erhitzten RPE-Melanosomen führen.

Die entstehenden Mikroblasen vergrößern dabei für µs das Zellvolumen und führen letztlich mit hoher Wahrscheinlichkeit zur Disruption und Desintegration der RPE-Zelle. Durch die Applikation von Mehrfachpulsen lässt sich die Bestrahlungsschwelle für Zellschädigung dabei stark senken.

Ein Lasersystem zur Augenbehandlung, das die mittlere Pulsenergie der emittierten Strahlung mittels Sensorik überwacht und durch Rückkopplung steuert, d.h. etwa auf einen vorab definierten Wert regelt, wird z.B. in der WO 91/19539 A1 beschrieben. Die beabsichtigte Schonung gesunder Zellen wird mit einem solchen System allerdings ebenso wenig sichergestellt wie seine therapeutische Wirksamkeit. Denn gerade bei der Behandlung des Augenhintergrunds unterscheiden sich die Voraussetzungen für die Lasertherapie (z.B. Transparenz von Linse oder Glaskörper, Pigmentierung der Retina) von Patient zu Patient erheblich.

Forschungsergebnisse zeigen, dass die benötigten Pulsenergien zur Erzeugung der RPE-Effekte schon intraindividuell um bis zu 100 %, interindividuell sogar deutlich stärker schwanken. Nach bisheriger Erkenntnis darf die Pulsenergie aber nicht mehr als einen Faktor zwei über der Schwellpulsenergie zur Erzeugung der RPE-Schäden liegen, da es ansonsten bereits zu sichtbaren Schäden an der Netzhaut kommt. Andererseits kann eine vorab getroffene Wahl der Laserenergie aus denselben Gründen auch zu gering sein, um das RPE überhaupt zu beeinflussen, was den Eingriff wirkungslos macht.

Eine Erfolgskontrolle der RPE-Schädigung kann heute unter klinischen Bedingungen erst nach Ende der Behandlung stattfinden. Zur Dosimetriekontrolle wird postoperativ eine Fluoreszenzangiographie des Augenhintergrunds vorgenommen. Den Patienten wird dazu ein Farbstoff (Fluoreszein oder ICG) in die Blutbahn injiziert, der von der Aderhaut durch das geschädigte RPE hindurch zur Netzhaut diffundiert und auf diese Weise die RPE-Defekte bei einer Fundusfluoreszenzphotographi,e demarkiert. Dieses invasive Verfahren ist aber in der Praxis niedergelassener Augenärzte nicht anwendbar und limitiert so zur Zeit die Therapie auf gut ausgerüstete Kliniken bzw. Universitätsaugenkliniken. Insbesondere kann bei mangelhaftem Behaadlungserfolg bis zum Abbau der Farbstoffe keine unmittelbare Fortführung der Behandlung erfolgen. Deren grundsätzlich giftige Wirkung erfordert überdies oft eine längere Therapiepause.

Wesentlich für eine wirksame und zugleich schonende Behandlung ist deshalb die kontinuierliche Beobachtung der Strahlungswirkung auf das Gewebe während des Eingriffs.

Die bereits beschriebene Zerstörung der RPE-Zellen durch thermische Disruption bildet den Ansatzpunkt der DE 199 32 477 A1 C2 zur Detektion der RPE-Schädigung anhand mechanischer Deformationen. Die kurzfristige Expansion bestrahlter RPE-Zellen bei der Bildung von Gasblasen (Mikrovaporisation), die unmittelbar mit der RPE-Zerstörung einhergeht, lässt sich hiernach akustisch messen und zur Steuerung eines Behandlungslasers verwenden. Unterhalb der zur Blasenbildung erforderlichen Pulsenergieschwelle ist die Materialantwort thermoelastisch und reproduzierbar, d.h. jeder eintreffende Puls erzeugt z.B. eine Druckwelle, die ausgehend vom Augenhintergrund den Augapfel durchläuft und als Funktion der Zeit mit einem Messwandler (z.B. ringförmige Piezokeramik in einem Kontaktglas) aufgezeichnet werden kann.

Zwei nacheinander applizierte identische Pulse erzeugen an derselben Materialstelle dabei praktisch identische Drucktransienten. Die Amplituden der Drucktransienten skalieren darüber hinaus näherungsweise linear mit der Pulsenergie, sofern diese unter der Schwelle der Blasenbildung bleibt.

Beim Überschreiten der Schwelle steigt die Druckamplitude hingegen überproportional an und der Verlauf der Transienten ändert sich von Puls zu Puls auch bei ansonsten gleich bleibender Pulsform und Pulsenergie der applizierten Strahlung aufgrund der Statistik des Einsetzens der Mikrovaporisation an den Melaningranula. Auf diese Weise lässt sich die therapeutisch wirksame Pulsenergie genau ermitteln, die auf RPE-Zellen wirkt und ihre Umgebung weitestgehend unversehrt lässt.

Mittlerweile haben erste Patientenbehandlungen gezeigt, dass sich die optoakustische Kontrolle prinzipiell sehr gut eignet, online direkt nach Applikation eines Laser-Bursts die Mikrovaporisation nachzuweisen. Nach bisheriger Erkenntnis korreliert das Ergebnis hervorragend mit den fluoreszenzangiographisch jeweils nach der Behandlung bestimmten RPE-Leckagen.

Die entstehenden akustischen Transienten werden mit Hilfe eines im Kontaktglas integrierten Schallwandlers gemessen. Dies beeinträchtigt die Behandlung nicht, da Kontaktgläser zur Kompensation der Lichtbrechung der Augenmedien ohnehin benötigt werden. Die akustischen Signale werden vorverstärkt, auf einen PC übertragen und verrechnet.

Das Ergebnis in Form einer einzigen Zahl wird direkt nach der Applikation des Bursts angezeigt und vermittelt dem Arzt sofort, ob die Pulsenergie für RPE-Effekte an der gerade behandelten Stelle ausreichend war. Falls nicht, kann sofort ein Burst mit höherer Pulsenergie nochmals appliziert werden.

Ein Hauptnachteil des soweit bestehenden Verfahrens liegt allerdings in seiner Umständlichkeit, da der Arzt eventuell mehrmals, mit steigender Pulsenergie, ein und dasselbe Areal bestrahlen muss, bis ein RPE-Effekt eintritt. Dabei ergibt sich zugleich das Problem der präzisen Position des Applikationsortes auf der Retina; wenn nämlich eine gewisse Zeit zwischen der Messung des Energiebedarfs und der tatsächlichen Therapiebestrahlung vergeht, können z.B. geringste Augenbewegungen bewirken, dass ein anderer als der vermessene Ort auf der Netzhaut therapiert wird. Aufgrund der bereits erwähnten Inhomogenität des Energiebedarfs zur RPE-Schädigung schon im Auge ein und desselben Patienten kann dies sowohl den Therapieerfolg als auch die Vermeidung von Kollateralschäden in Frage stellen. Zudem läßt sich das bestrahlte Areal auf der Netzhaut nicht ein zweites Mal bestrahlen, da der Ort der Strahlungsapplikation für den Arzt nicht sichtbar ist, unabhängig von dem Erfolg der Bestrahlung, da ein Auge nie länger als wenige Sekunden unbewegt bleibt.

Die optische Erfassung von Mikrovaporisation an der Netzhaut ist in WO 01/91661 A1 dargestellt, wobei die Rückreflektion von Behandlungs- oder Probelaserlicht ausgenutzt wird, die sich bei einsetzender Blasenbildung erhöht.

Weder in WO01/91661 A1 noch in der bereits genannten DE 199 32 477 A1 sind Algorithmen oder Hinweise zur Steuerung des Behandlungslasers aufgeführt, die es ermöglichen, an allen Orten der Retina, bei zum Teil stark schwankenden Schwellwerten für RPE-Effekte schwellennah zu behandeln. Es hat sich bereits klinisch gezeigt, dass die schwellennahe Bestrahlung für selektive RPE-Effekte unbedingt erforderlich ist, da es ansonsten zu Schädigungen an der Netzhaut kommt.

Erste Ergebnisse von Untersuchungen des Erfinders zeigten an einzelnen Patienten Behandlungsenergieschwellen zwischen 200 und 350 µJ pro Puls, die Schwellen zur Netzhautschädigung (optische Sichtbarkeit der Effekte) lagen bei 300 µJ an den Arealen mit niedriger Schwelle. Das heißt Pulsenergie, die an bestimmten Arealen zu keinen Effekten führen, resultieren in anderen Arealen bereits in sichtbaren Netzhautschäden.

Es ist deshalb erwünscht, eine Steuerung des Behandlungslasers so vorzunehmen, dass eine Bestrahlung sehr dicht oberhalb der Schwelle für RPE-Schäden möglich ist, zur Minimierung des Risikos schwerwiegender Nebenwirkungen. Dabei soll eine mehrmalige Strahlungs-Applikation pro Bestrahlungsort vermieden werden. Der Arzt soll nur einmal applizieren müssen und sich sicher sein können, dass die gewollten RPE-Effekte eintreten.

Aufgabe der Erfindung ist demzufolge, die Steuerung eines Bestrahlungslasers anzugeben, bei dem Laserpulsfolgen oder sich verändernde längere Pulse während des Applizierens dergestalt modifiziert werden, daß die Vergleichbarkeit erfaßter Transienten gewahrt bleibt.

Die Aufgabe wird gelöst durch Laser mit den Merkmalen des Hauptansprüche. Die Unteransprüche geben vorteilhafte Ausgestaltungen der Erfindung an.

Die Arbeit des Aufsuchens einer therapeutisch wirksamen Pulsenergie braucht nicht mehr separat durchgeführt werden. Von einer Effektivität der Behandlung kann zuverlässig ausgegangen werden. Dies ist insbesondere wesentlich für die Verbreitung solcher Behandlungslaser in jede Augenarztpraxis, wo die oben genannten Methoden zur Erfolgskontrolle nicht oder nur mit großem Aufwand durchgeführt werden könnten bzw. ansonsten eine spezielle Ausbildung des Arztes zur Laserregelung erforderlich wäre.

Erfindungsgemäß wird weiter eine Steuerung bereitgestellt, die die zentralen Eckpunkte für ein maschinenimplementierbares Programm zur Steuerung des Lasers formuliert. Es wird vorteiilhafterweise ein Laser mit Dosimetriesteuerung verwendet, der eine Einrichtung zur Erfassung einer thermisch induzierten Blasenbildung in bestrahlten Gewebe und zur Nutzung des Signals zur Steuerung des Lasers aufweist. Die Einrichtung zur Erfassung der Blasenbildung kann ein Druckwandler sein oder ein piezokeramischer Drucksensor wird als Einrichtung zur Erfassung von Drucktransienten vorgesehen. Es ist aber auch möglich, dass die Einrichtung zur Erfassung der Blasenbildung ein Photodetektor ist.

Besonders Laser mit einem integrierten an das ophthalmologische Applikationsinstrument adaptierten, kompakten Faserinterferometer eignen sich.

Eine im Laserstrahlverlauf angeordnete Abschwächungseinheit zur schnellen Verringerung der auf bestrahltes Gewebe aufgebrachten Laserleistung ermöglicht vergleichbare Verhältnisse bei den erfaßten Transienten.

Um nun eine Steuerung zum Betreiben eines Lasers zur Applikation einer Pulsfolge oder eines langen Pulses mit sich stufenweise erhöhender Leistung anzugeben, muß gleichzeitig eine Erfassung von etwaiger Mikrovaporisation am bestrahlten Gewebe, d.h. Detektion der Blasenbildung erfolgen, die sich auf die Applikation wenigstens einer weiteren, gegenüber der ersten veränderten Pulsfolge, nachdem erstmals Blasenbildung festgestellt wurde, auswirkt. Dabei sollen die Teilsequenzen oder Einzelpulse je eine Dauer zwischen 0,05 und 50 Mikrosekunden besitzen und die applizierte Leistung durch Variation der Dauer der Einzelpulse oder der Folgerate der Einzelpulse geregelt werden.

Dabei ergeben sich die zwei Möglichkeiten zur Bestrahlung eines Gebietes 1) Betreiben eines Lasers durch Applizieren eines langen das gesamte Gebiet abdeckenden Pulses steigender Leistung bei gleichzeitigem Erfassen von etwaiger Mikrovaporisation am bestrahlten Gewebe, und Beenden des Pulses mit anderer Leistung sobald erstmals Blasenbildung festgestellt wird und 2) Applizieren eines andauernden Laserstrahls, der mittels Ablenkeinrichtungen ein Abtastmuster jeweils nur ein Teilgebiet der Gewebefläche abdeckend durchführt, bei gleichzeitigem Erfassen von Mikrovaporisation am bestrahlten Gewebe, und Modulation der Leistung des Lasers derart, dass größtenteils dicht oberhalb der Blasenbildungsschwelle appliziert wird. Möglichkeit 2 ist nicht Teil dieser Erfindung.

Zur Erfassung der Blasenbildung und der einhergehenden Änderung der Reflektivität des Gewebes interferometrisch über eine Veränderung optischer Weglängen (Brechungsindex) erfasst wird.

Dabei kann durch Beleuchtung des untersuchten Gewebes mit einer leistungsschwachen Probelichtquelle, insbesondere eines Lasers, der eine wesentlich andere Wellenlänge als die der therapeutisch wirksamen Laserpulsfolge aufweist, wobei wenigstens ein Farbfilter vor dem Detektor die Erfassung von Behandlungslichtwellenlängen unterdrückt.

Dieser Probelichtstrahl wird dem Behandlungslichtstrahl vorteilhafterweise nachgeführt

Erfindungsgemäß wird nun vorgeschlagen, bei der Steuerung zum Betreiben eines Bestrahlungslasers, zunächst Laserpulsfolgen endlicher Dauer ("Bursts"), jeweils umfassend wenigstens zwei zeitlich nacheinander applizierte Teilpulsfolgen umfassend mindestens je einen Puls zu applizieren, wobei eine erste Teilpulsfolge ausgehend von einem vorbestimmten geringen Pulsenergieniveau monoton steigende Pulsenergie aufweist, und wobei eine zweite Teilpulsfolge im wesentlichen konstante Pulsenergie im Bereich des Endwertes der ersten Pulsfolge aufweist; dabei etwaige Blasenbildung am Bestrahlungsort während des Applizierens der ersten Teilpulsfolge zu erfassen, und die erste Pulsfolge zu beenden und die zweite Pulsfolge zu beginnen, sobald Blasenbildung im Gewebe festgestellt wird.

Es können aber auch Teilpulsfolgen verwandt werden, so daß etwa mit drei Teilpulsfolgen endlicher Dauer, jeweils umfassend wenigstens drei zeitlich nacheinander applizierte Teilpulsfolgen umfassend mindestens je einen Puls gearbeit wird, wobei die erste Teilpulsfolge konstante Pulsenergie unterhalb der zur Schädigung des biologischen Gewebes benötigten Pulsenergieschwelle aufweist, die zweite Teilpulsfolge ausgehend vom Pulsenergieniveau der ersten Teilpulsfolge monoton steigende Pulsenergie aufweist, die dritte Teilpulsfolge im Wesentlichen konstante Pulsenergie im Bereich des Endwertes der zweiten Pulsfolge aufweist, und etwaige Blasenbildung im biologischen Gewebe am Bestrahlungsort während des Applizierens der ersten und zweiten Teilpulsfolge erfaßt wird, so daß eine Beendigung der zweiten Pulsfolge und der Begin der dritten Pulsfolge erfolgt, sobald erstmals Blasenbildung im Gewebe festgestellt wird.

Diese dritte Teilpulsfolge kann eine Mehrzahl von Pulsen umfassen, deren Pulsenergie geringfügig unterhalb des Endwertes der zweiten Teilpulsfolge liegt.

Alternativ können die Teilpulsfolge eine zunächst konstante Pulsrate derart aufweisen, dass die pro Zeiteinheit applizierte Gesamtenergie nicht zur Schädigung des biologischen Gewebes ausreicht, die zweite Teilpulsfolge ausgehend vom Pulsratenniveau der ersten Teilpulsfolge eine monoton steigende Pulsrate aufweist, und die dritte Teilpulsfolge im Wesentlichen eine Pulsrate im Bereich des Endwertes der zweiten Pulsfolge aufweist, so daß ein Erfassen etwaiger Blasenbildung im biologischen Gewebe am Bestrahlungsort während des Applizierens der ersten und zweiten Teilpulsfolge, und ein Beenden der zweiten Pulsfolge und Beginnen der dritten Pulsfolge erfolgt, sobald erstmals Blasenbildung im Gewebe festgestellt wird.

Dabei sollte ein die Blasenbildung indizierendes Messsignal während der ersten Teilpulsfolge für jeden Bestrahlungsort neu kalibriert werden, und die Laserpulsfolgen vorteilhafterweise Pulsdauern zwischen 0,05 und 50 Mikrosekunden aufweisen.

Aber auch das Applizieren eines kontinuierlich emittierenden Laserstrahls, der über einen steuerbaren Deflektor auf eine Vielzahl von Bestrahlungsorten auf dem biologischen Gewebe nacheinander gelenkt wird, wobei sich die Ablenkung des Laserstrahls periodisch wiederholt ist möglich, wenn etwaiger Blasenbildung im biologischen Gewebe an den Bestrahlungsorten, während die Laserleistung monoton erhöht wird, erfaßt wird, und eine Reduktion der Laserleistung nach einer vorgegebenen Wirkzeit, die beginnt, sobald erstmals Blasenbildung im Gewebe festgestellt wird, erfolgt.

Dabei sollte die emittierte Laserleistung resonatorextern abgeschwächt bzw. durch Rücknahme der Abschwächung gesteigert werden. Diese Abschwächung kann z.B. durch das Ansteuern eines Polarisators erfolgen.

Die Steuerung zum Betreiben des biologisches Gewebe bestrahlenden Lasers kann auch durch Applizieren eines einzelnen Laserpulses von bis zu 50 Mikrosekunden Pulsdauer, dessen Pulsenergie während einer ersten Zeitspanne seines Aussendens monoton ansteigt und während einer zweiten Zeitspanne seines Aussendens im Wesentlichen konstant bleibt, Erfassen etwaiger Blasenbildung im biologischen Gewebe am Bestrahlungsort während der ersten Zeitspanne des Aussendens des Laserpulses, und Abbrechen des Laserpulses nach einer vorgegebenen Wirkzeit, die beginnt, sobald erstmals Blasenbildung im Gewebe festgestellt wird, angegeben werden.

Dabei sollte der Laserpuls mit einem gütegeschalteten Laser erzeugt werden, der eine Pulsformkontrolle erlaubt, damit auch eine Blasenbildung im Gewebe durch das Aufzeichnen von durch das Gewebe laufenden Drucktransienten, die vom Ort der Blasenbildung ausgehen, oder eine einhergehende Änderung der Reflektivität des Gewebes erfasst werden kann. Die Blasenbildung kann aber auch interferometrisch über eine Veränderung optischer Weglängen (Brechungsindex) erfaßt werden, wobei dies durch Beleuchtung des untersuchten Gewebes mit einer leistungsschwachen Probelichtquelle, insbesondere einem Laser, der eine wesentlich andere Wellenlänge als die des therapeutisch wirksamen Lasers aufweist erfolgen kann. Ein Farbfilter vor dem Detektor sollte dabei die Erfassung von Behandlungslichtwellenlängen unterdrücken.

Vorgeschlagen wird, daß das Probelicht mit demselben Deflektor wie das Behandlungslicht derart abgelenkt wird, dass der Beleuchtungsort des Probelichts dem Bestrahlungsort des Behandlungslichts folgt.

Weitere Vorteile und Merkmale der Erfindung werden anhand der Vorgehensweise und anhand der Zeichnung erläutert. Dabei zeigt :
Fig. 1 eine Übersichtsskizze eines Ausfilhrungsbeispiels für eine optoakustische Messung an einem Auge,
Fig. 2 Meßergebnisse in Kurvenform die die Superposition der Drucktransienten unterhalb (A) und oberhalb (B) der Schwellenergie zur Blasenbildung verdeutlichen, wobei Zeit auf der x-Achse und Druck in willkürlichen Einheiten auf der y-Achse aufgetragen sind (jeweils dreißig Spuren),
Fig. 3a ein Beispiel für eine ansteigende Pulsenergiefolge
Fig. 3b ein weiteres Beispiel für eine ansteigende Pulsenergiefolge
Fig. 3c ein Beispiel eines nicht weiter Anhebens der Pulsenergie ab Detektion,
Fig. 3e ein Beispiel für die Veränderung des Impulsabstandes,
Fig. 3f ein Beispiel einer Ein-Puls-Bestrahlung,
Fig. 4 eine schematische Darstellung der Verfahrensschritte.

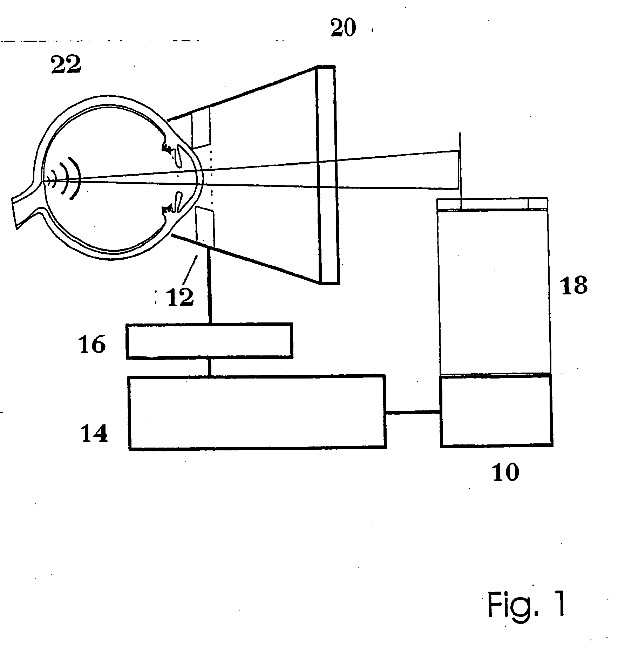

Aus Fig. 1 ast ersichtlich, dass das ophthalmologische Behandlungssystem im Prinzip aus drei Komponenten besteht: aus einem Laser 10, aus einer Detektionseinheit 12 zur Messung der Bestrahlungswirkung im Gewebe sowie aus einer Auswerte- und Steuereinheit, die wenigstens einen programmierbaren Mikroprozessor 14, insbesondere-einen PC, und gewöhnlich auch einen Verstärker 16 für die Detektorsignale umfasst. Bezugszeichen 18 steht für eine Schlitzlampe, 20 für eine optoakustische Kontaktlinse und 20 für das zu behandelnde Auge.

Konkret ist in Fig. 1 die Messung von Drucktransienten mit einem Piezodetektor 12 aus PZT (Bleizirkoniumtitanat) vorgesehen, weil für diesen Aufbau bereits gesicherte Erkenntnisse vorliegen.

Im Prinzip ist eine Vielzahl alternativer Verfahren zum Nachweis von Blasenbildung im bestrahlten Gewebe denkbar, wobei besonders rein optische zu bevorzugen sind. So ändert sich beispielsweise bei der Blasenbildung die Reflektivität des Gewebes erheblich, wenn zusätzliche Grenzflächen Flüssigkeit/Gasphase erstmals in Erscheinung treten. Dies kann durch direkte Messung der reflektierten Lichtintensität festgestellt werden, wobei man vorzugsweise einen leistungsschwachen Probelaser parallel zum Behandlungslaser einsetzen würde. Beide Laser sollten bevorzugt unterschiedliche Wellenlängen emittieren, so dass mittels eines farbselektiven Filters nur das Probelicht, das selbst möglichst wenig vom Gewebe absorbiert wird, zur Detektionseinheit (Photorezeptor, z.B. CCD) gelangt.

Eine weitere Möglichkeit ist die interferometrische Messung der Blasenbildung, bei der die Laufzeit des Laserlichts innerhalb der RPE-Zellschicht ermittelt wird. Das Auftreten von Gasblasen ändert den Brechungsindex der Schicht besonders deutlich. Von den beiden Grenzflächen der typisch 300 µm dicken RPE-Schicht rückgestreutes Licht besitzt einen Gangunterschied, dessen Änderung mit einem Interferometer festgestellt werden kann.

Im folgenden beschränkt sich die Erläuterung der Erfindung auf die optoakustische Gewebeantwort, d.h. auf aufnehmbare Drucktransienten.

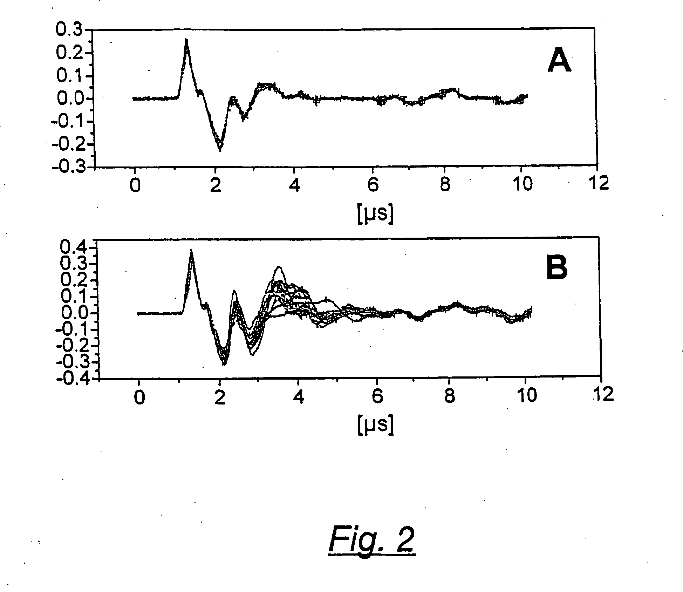

Fig. 2 (A) und (B) zeigen je die Überlagerung von 30 aufgezeichneten Drucktransienten für 30 gleichförmige Laserpulse, die während eines Laserbursts emittiert werden. Liegt die Pulsenergie unter der Blasenbildungsschwelle, so zeigen alle Transienten praktisch identischen Verlauf (A). Ursache ist die thermoelastische Expansion des Gewebes unter Bestrahlung, wobei das Gewebe nach Pulsende offenbar in seinen Ausgangszustand zurückkehrt. Der Transientenverlauf ändert sich auch dann nicht wesentlich, wenn höhere Pulsenergien -immer noch unterhalb der Schwelle-benutzt werden.

Es empfiehlt sich, die gemessenen Amplituden der Transienten jeweils durch die Pulsenergie zu teilen und so das elektrische Messsignal für verschiedene Pulsenergien zu normieren. Reicht die Pulsenergie hingegen zur Blasenbildung aus, bewirkt der statistische Charakter der Verdampfung einen jeweils anderen Verlauf der Transienten von Puls zu Puls, auch dann, wenn die Pulse stets dieselben Merkmale besitzen (B). Aus dieser Eigenschaft des Ensembles der Messsignale kann mittels einer gängigen Rechneranalyse nach dem Burst festgestellt werden, ob eine RPE-Schädigung durch thermische Disruption eingetreten ist oder nicht.

Um nun mehrfaches Applizieren der Laserstrahlung auf dieselbe Stelle der Netzhaut überflüssig zu machen, wird das erfindungsgemäße Lasersysteme dafür ausgelegt, die Pulsenergie innerhalb eines einzigen Bursts zu verändern, im wesentlichen zu steigern. Mit Hilfe der oben genannten Normierung der Signale kann der Signalvergleich für aufeinander folgende Pulse unterschiedlicher Energie auch in Echtzeit kontinuierlich durchgeführt und zur Entscheidung benutzt werden, ob der nächste Puls eine etwas höhere Energie besitzen soll oder nicht. Die benötigte Geschwindigkeit der Signalverarbeitung stellt mit heutigen GHz-Prozessoren keine große Herausforderung mehr dar.

Das Variieren der Pulsenergie lässt sich bevorzugt durch resonatorexterne Abschwächung des Behandlungslichts in einer Abschwächungseinheit realisieren. So kann die in der Regel polarisierte Laserstrahlung durch einen zweiten von der Steuereinheit verstellbaren Polarisator, dem ein fester Polarisationstrahlteiler folgt, kontrolliert abgedunkelt werden. Elektrisch schaltbare Polarisatoren mit ausreichender Schaltgeschwindigkeit und Präzision sind verfügbar, z.B. Pockelszellen. Auch akusto-optische Modulatoren, die polarisationsunabhängig arbeiten, sind einsetzbar.

Es ist hier zu betonen, dass das Verändern der Pulsenergie durch eine Regelung der Pumpleistung nachteilig ist, da sie in der Regel zugleich die Pulsform beeinflusst, was nur durch aufwendige Elektronik vermeidbar ist. Bei Änderung der Pulsform werden auch die Drucktransienten jeweils andere Verläufe zeigen, selbst wenn keine Blasenbildung stattfindet.

Es sind eine Reihe von Strategien denkbar, in welcher Weise die Pulsenergie dem Messbefund (Mikrovaporisation: ja oder nein) anzupassen ist. Der Einfachheit halber wird im Weiteren davon ausgegangen, dass Signalbewertung und Energieregelung jeweils zwischen zwei Einzelpulsen stattfinden. Dies ist sicherlich eine bevorzugte Ausführung des Verfahrens, doch können im Prinzip anstelle der Einzelpulse auch mehrpulsige Teilsequenzen des gesamten Laserbursts pro Anpassungszyklus berücksichtigt werden.

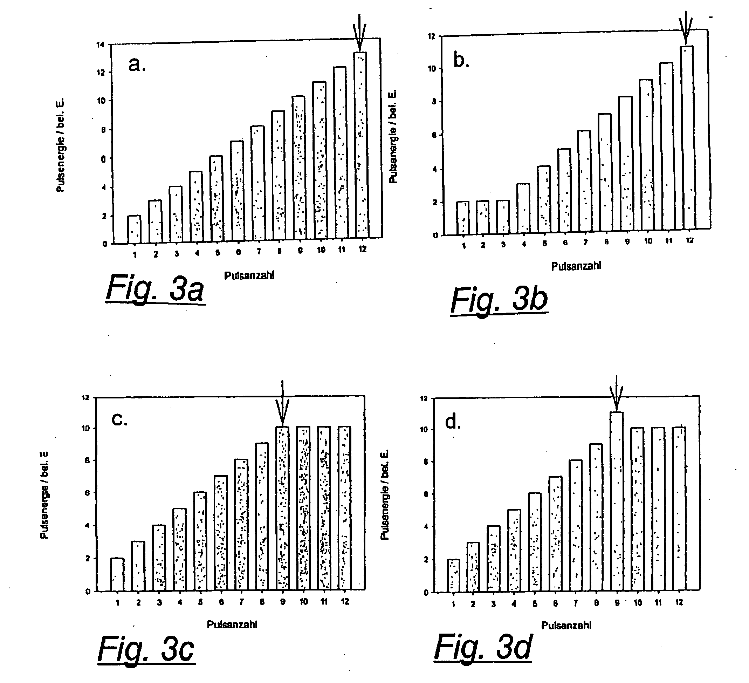

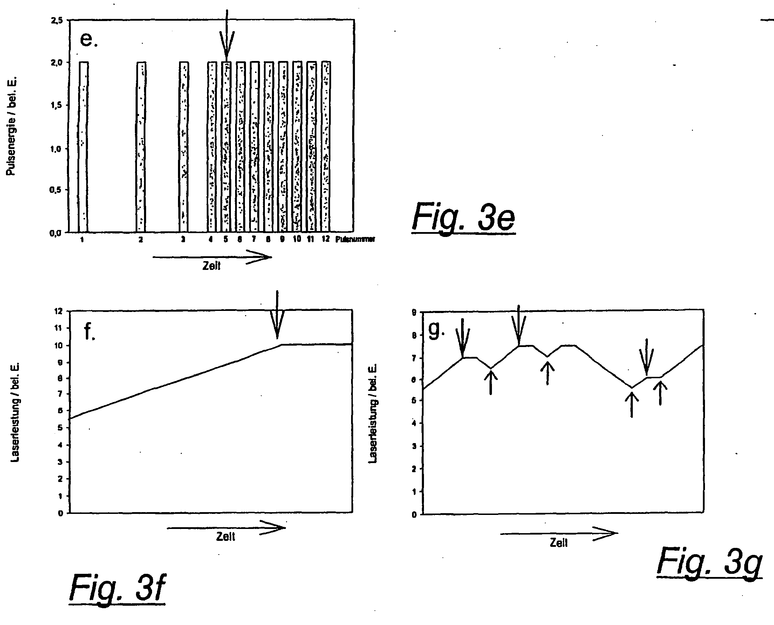

Fig. 3 stellt exemplarisch eine Auswahl von Regelstrategien grafisch dar, Fig. 3a-e jeweils für repetierend gepulste Bestrahlung, Fig. 3f für eine Ein-Puls-Bestrahlung, Fig. 3g für eine scannende Anwendung. Der Pfeil nach unten (□) in den Abbildungen markiert jeweils den Puls, bei dem die Blasenbildung nachgewiesen wird, der Pfeil nach oben (□) den Zeitpunkt wo keine Messung der Mikroblasen mehr erfolgt.

Allen Strategien ist gemein, dass eine monoton ansteigende Rampe der Laserleistung verfolgt wird, die erst endet, sobald Mikrovaporisation auftritt. Beispielsweise wird bei Strategie A nach dem Erkennen der Verdampfung noch ein weiterer Puls etwas höherer Energie appliziert, um sicher therapeutische Wirkung zu erzielen. Der Burst wird danach abgebrochen.

Strategie B beginnt mit einer multiplen Pulssequenz niedriger Energie, um ein besseres Grundsignal unterhalb der Blasenschwelle aufzufinden, aus dem sich die Schwellenergie möglichst zuverlässig ableiten lässt. Der Burst endet konservativ beim ersten Anzeichen von Gasblasen, d.h. Strategie B ist die der maximalen Vorsicht.

Strategie C ist demgegenüber maximal wirksam, indem man dem ersten Puls mit Mikrovaporisation gleich eine Reihe weiterer mit derselben Energie hinterherschickt.

Strategie D ist hier etwas rücksichtsvoller, indem die Pulsenergie nach dem Schwellpuls (Pfeil) geringfügig reduziert wird. Dies entspricht der Erkenntnis, dass bei repetierender Bestrahlung therapeutische Schäden mit kleineren Pulsenergien zu erzielen sind als bei Einzelpulsen. wird. Wird diese erreicht, hält man die Pulsfolgerate konstant. Diese Lösung zeichnet sich besonders durch ihre einfache Implementierung aus, da lediglich der Frequenzgeber angesteuert werden muss und keine schnelle Hochspannungselektronik nötig ist. In Messungen zeigte sich, dass die Pulsform und Energie bis zu Pulsfolgeraten von 500 Hz unverändert bleibt.

Die geschilderten Strategien lassen sich alle ohne Aufwand in die Mikroelektronik der Steuereinheit implementieren und können dort auch parallel als wählbare Optionen gespeichert vorliegen, die per Knopfdruck durch den Arzt aufgerufen werden.

Weitere Fortbildungen können in der Art der Leistungssteigerung liegen, die in Fig. 3a-d beispielhaft linear gewählt ist. Auch eine geometrische Progression (z.B. Anstieg um jeweils 5 %) der Leistung oder irgendeine andere Wachstumsvorschrift kommen in Frage, solange zugleich das deutliche Überschreiten der Schwellenergie vermieden wird.

Bei Umsetzung der Strategien C oder D kann es zudem sinnvoll sein, die Anzahl der therapeutisch wirksamen Pulse nach Erreichen der Verdampfutigsschwelle bei jedem Burst gleich einzurichten, insbesondere einer vorab programmierten Vorgabe durch den behandelnden Arzt folgende.

Die bevorzugte Ausfuhrungsform des gesteuerten Lasersystems umfasst einen gütegeschalteten Laser, der bei Aktivierung eine Pulsfolge variabler Pulsenergie mit wenigen Mikrosekunden Pulsdauer emittiert. Eine halbe Mikrosekunde entspricht ungefähr der thermischen Relaxationszeit der bestrahlten Melanosomen, 50 µs etwa der des RPE- d.h. die durch Bestrahlung erzeugte lokale Erwärmung in kürzeren Applikationszeiten als der Relaxationszeit zieht keine signifikant erhöhten Temperaturen nach sich, die zu einer Schädigung angrenzenden Gewebes führen könnten.

Demnach kann auch ein langer Einzelpuls, z.B. von 50 µs Pulsdauer, den schonenden Therapieeffekt erzielen, wenn die Pulsleistung erst im Verlauf seines Aussendens den obigen Strategien entsprechend ansteigt (Fig. 3f). Wichtig ist, den langen Puls kurz nach dem Eintreten von Blasenbildung abzuschalten. Die Energierampe wird dann bei jedem Einzelpuls erneut durchfahren. Zur jeweiligen Detektion der Mikrovaporisation sollte bevorzugt auf den optischen Nachweis zurückgegriffen werden.

Eine weitere Ausfürungsform nicht Teil dieser Erfindung umfasst einen cw-(engl. Abkürzung für continuous wave, einen fortlaufend emittierenden)-Laser, dessen Licht mit Hilfe eines optoakustischen Deflektors kontrolliert abgelenkt und scannend über die gesamte Netzhaut geführt wird. Die Abtastrate des Scanners bestimmt hier die Repetitionsrate des "quasi-gepulsten" Lichts auf jedem Punkt der Retina. Das Licht eines zweiten, leistungsschwachen Probelasers wird durch den Modulator so abgelenkt, dass sein Auftreffpunkt auf der Netzhaut dem des Behandlungslasers unmittelbar folgt. Die Leistung des Behandlungslichts wird während des Scans gesteigert, bis sich anhand des rückgestreuten Behandlung- oder Probelichts Blasenbildung erkennen lässt. Die Behandlungsleistung wird dann nach kurzer Zeit wieder abgesenkt, bis keine Blasenbildung mehr detektiert wird und der Rampenanstieg beginnt von vom.

Eine mögliche Strategie hierzu ist in Abb. 3g aufgezeigt. Die Pulsleistung am Ausgang des Behandlungslasers entspricht somit einer durch Rückkopplung modulierten, unregelmäßigen Sägezahn-Funktion. Selbstverständlich sind auch zahlreiche weitere Variationen bei diesem Aufbau denkbar.

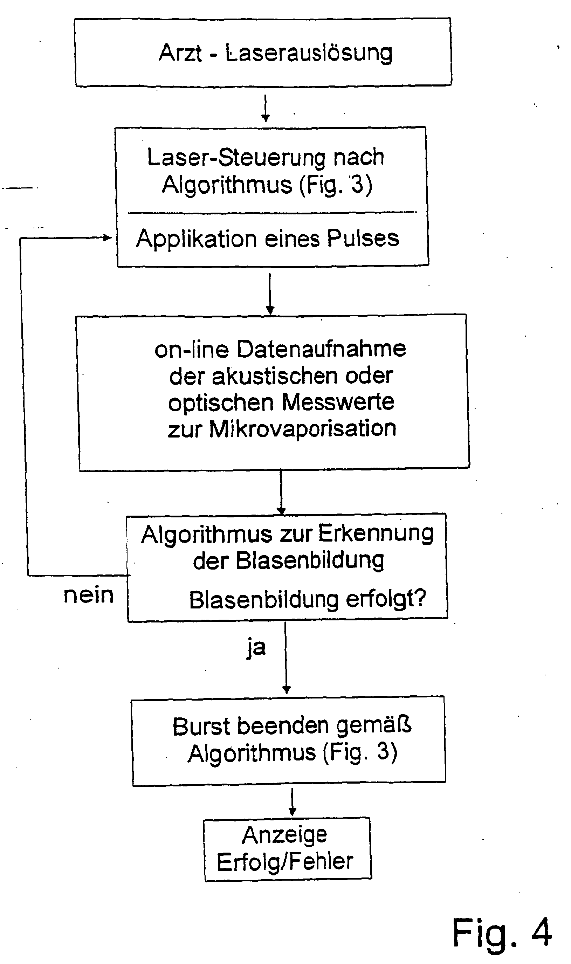

Das vorgestellte Verfahren garantiert durch das messtechnische Erfassen von Gewebeverdampfung, dass bei der lasergestützten Thermotherapie beabsichtigte Gewebeschäden, insbesondere am Augenhintergrund, beim Applizieren gepulster Laserstrahlung eintreten. Zugleich ist durch die vorgeschlagene Regelvorschrift am Beispiel für repetierend gepulste Bestrahlung (Flussdiagramm Fig. 4) sichergestellt, dass das Lasersystem automatisch die minimal notwendige Laserleistung aufsucht und anwendet. Ähnliche Algorithmen sind auch für Einzelpuls oder scannende Bestrahlung anwendbar.

In der Anwendung auf das Retinale Pigmentepithel (RPE) kann hierdurch Schaden an nicht zu behandelnden Zellen, insbesondere an Photorezeptoren, ohne das aktive Eingreifen des Arztes zwecks Dosimetriekontrolle vermieden werden.

Der verwendete Laser ist demzufolge mit einer Dosiemetriesteuerung versehen zur Abgabe von zeitlich begrenztem Laserlicht gesteuert durch eine Einrichtung zur Erfassung einer thermisch induzierten Bläschenbildung und den einhergehenden Dichteschwankungen und Reflektivitätsänderungen im bestrahlten Gewebe. Diese Dichteschwankung kann durch einen Schallwandler optoakustisch erfaßt werden. Ein piezokeramischer Drucksensors ist besonders geeignet. Zur Erfassung der Dichteschwankungen, die die Reflektivität des Gewebes verändern, kann zum einen die Änderung der Intensität (Brechungsindexdurch einen Schall wandler optoakustisch erfaßt werden. Ein piezokeramischer Drucksensors ist besonders geeignet. Zur Erfassung der Dichteschwankungen, die die Reflektivität des Gewebes verändern, kann zum einen die Änderung der Intensität (Brechungsindexsprung) direkt mit einer Photodiode erfasst und ausgewertet werden. Zum anderen kann das Aufschwingen der Blase über eine Veränderung optischer Weglängen interferometrisch erfasst werden, was den Vorteil der Streulichtunterdrückung aus dem Auge bietet und Zusatzinformationen über die Größe und Ausdehnung der Blase ergibt.

Zur schnellen Steuerung der Leistung ist im Laserstrahlverlauf bevorzugt eine Strahlabschwächungseinheit insbesondere zur schnellen Verringerung der auf bestrahltes Gewebe aufgebrachten Laserleistung angeordnet. Alternativ lässt sich bei repetierender Bestrahlung die applizierte mittlere Leistung über die Pulsfolgerate modulieren.

Das erfindungsgemäße Verfahren zum Betreiben eines gepulsten Lasers stellt sich dann wie folgt dar: Applizieren von Pulsfolgen mit sich stufenweise erhöhender Leistung und gleichzeitiges Erfassen von etwaiger Mikrovaporisation am bestrahlten Gewebe und Applizieren weiterer Pulsfolgen im Wesentlichen mit derselben Leistung, bei der erstmals Blasenbildung festgestellt wird. Dabei sind bevorzugt alle Teilsequenzen der Pulsfolge gleich lang und bestehen vorzugsweise aus einzelnen Pulsen, wobei die Teilsequenzen oder Einzelpulse je eine Dauer zwischen 50 Nanosekunden und 50 Mikrosekunden besitzen.

Der Laser mit Dosimetriesteuerung kann zur Erfassung der Blasenbildung auch mit einem ein Photodetektor versehen sein oder es können sogar eine Detektorzeile und Lichtleiter zum Aufbau eines integrierten Interferometers vorgesehen werden. Bevorzugt sind hierfür kompakte, an das ophthalmologische Applikationsinstrument adaptierte Faserinterferometer geeignet.

Bei Beleuchtung des untersuchten Gewebes zur Detektion der Blasenbildung mit einer leistungsschwachen Probelichtquelle, insbesondere eines Lasers, der eine wesentlich andere Wellenlänge als die der therapeutisch wirksamen Laserpulsfolge aufweist, kann dabei über ein Farbfilter vor dem Detektor die Erfassung von Behandlungslichtwellenlängen unterdrückt werden.

Weiter kann statt einer Pulsfolge auch ein langer Puls mit kontinuierlich steigender Leistung bei gleichzeitigem Erfassen von etwaiger Mikrovaporisation am bestrahlten Gewebe verwandt werden, wobei nach Feststellen der Mikrovaporisation der Puls beendet wird.

Femer-kann ein cw-Laser, dessen Licht mit Hilfe z.B. eines optoakustischen Deflektors kontrolliert abgelenkt und scanned über die gesamte Netzhaut geführt wird, benutzt werden, wobei die abgegebene Leistung im wesentlichen sägezahnartig variiert wird. Die Mikrovaporisation kann dabei vorzugsweise optisch anhand reflektierten Lichts des Behandlungslasers oder eines in der Wellenlänge anderen Probelasers erfolgen, welcher das die Mikrovaporisation betreffende Areal beleuchtet.

## Patentansprüche

1. Laser zur Bestrahlung biologischen Gewebes in einem Eingriff bei kontinuierlicher Online-Beobachtung der Stralitungswirkung auf die Gewebe während des Eingriffs mit:
- einer Steuerung zum Emittieren von Laserpulsfolgen endlicher Dauer in Form von Bursts, jeweils umfassend :
wenigstens zwei zeitlich nacheinander applizierte Teilpulsfolgen umfassend mindestens je einen Puls,
wobei eine erste Teilpulsfolge ausgehend von einem vorbestimmten geringen Pulsenergieniveau monoton steigende Laserleistung aufweist, und wobei eine zweite Teilpulsfolge im wesentlichen konstante Laserleistung im Bereich des Endwertes der ersten Pulsfolge aufweist,
wobei die aus zwei Teilpulsfolgen bestehenden Laserpulsfolgen Einzelpulse mit einem sich aus der Pulsfolgerate ergebenden Abstand mit Pulsdauern zwischen 0,05 und 50 µs aufweisen,
- einem Detektor zur Feststellung etwaiger Blasenbildung am Bestrahlungsort während des Emittierens der ersten Teilpulsfolge.
wobei die Steuerung zur Beendigung der ersten Pulsfolge und zum Beginnen der zweiten Pulsfolge geschaltet ist, so daß keine Suche einer therapeutisch wirksamen Pulsenergie mehr separat durchgeführt werden muss, sobald von dem Detektor ein Signal angelegt ist, dass indikativ für Blasenbildung im Gewebe ist.

2. Laser nach Anspruch 1, **dadurch gekennzeichnet, dass** die Steuerung zur Abgabe einer zweiten Teilpulsfolge mit einer Mehrzahl von Pulsen eingerichtet ist, deren Laserleistung geringfügig unterhalb des Endwertes der ersten Teilpulsfolge liegt.

3. Laser nach Anspruch 1, **gekennzeichnet durch** eine außerhalb des Resonators vorgesehene steuerbare Abschwächungseinheit für die emittierter Laserstrahlung, um diese einer Regelung zu unterwerfen.

4. Laser nach Anspruch 3, **gekennzeichnet durch** Mittel zur Pulsformkontrolle vorbestimmter Laserpulse, die an einem gütegeschalteten Laser vorgesehen sind.

5. Laser nach Ansprüchen 3 oder 4, **gekennzeichnet durch** Phasenänderung-erfassende Mittel des Detektors zur Erfassung der Blasenbildung, die mit einer Phasenänderung einhergeht.

6. Laser nach Ansprüchen 3 bis 5, **gekennzeichnet durch** interferometrische Mittel des Detektors zur Erfassung der Blasenbildung, die die bei Blasenbildung erfolgende Veränderung optischer Weglängen erfassen.

7. Laser nach Ansprüche 3 bis 5, **gekennzeichnet durch** eine Photodiode des Detektors zur Erfassung der Blasenbildung, die die bei Blasenbildung erfolgende Veränderung des Brechungsindex erfasst.

8. Laser zur Bestrahlung biologischen Gewebes in einem Eingriff bei kontinuierlicher Online-Beobachtung der Strahlungswirkung auf die Gewebe während des Eingriffs mit:
- einer Steuerung zum Emittieren eines einzelnen Laserpulses von bis zu 50 Mikrosekunden Pulsdauer, dessen Pulsenergie während einer ersten Zeitspanne seines Aussendens monoton ansteigt und während einer zweiten Zeitspanne seines Aussendens im wesentlichen konstant bleibt,
- einem Detektor zur Feststellung etwaiger Blasenbildung im biologischen Gewebe am Bestrahlungsort während der ersten Zeitspanne des Aussendens des Laserpulses,
- wobei die Steuerung zur Beendigung der ersten Zeitspanne und zum Beginn der zweiten Zeitspanne geschaltet ist, sobald von dem Detektor ein Signal angelegt ist, dass indikativ für Blasenbildung im Gewebe ist, und zur Beendigung des Laserpulses nach einer vorgegebenen Wirkzeit, die beginnt, sobald erstmals Blasenbildung im Gewebe festgestellt wird, so dass keine Suche einer therapeutisch wirksamen Pulsenergie mehr separat durchgeführt werden muss.

## Claims

1. Laser for irradiating biological tissue during surgery with continuous online monitoring of the radiation effect on the tissues during the surgery, having:
- a control for emitting laser pulse sequences of finite duration in the form of bursts, each comprising:
at least two temporally-sequentially applied part-pulse sequences comprising at least one pulse each,
from a predetermined low pulse energy level, a first part-pulse sequence having a monotonously increasing laser power and
a second part-pulse sequence having an essentially constant laser power in the range of the end value of the first pulse sequence,
the laser pulse sequences consisting of two part-pulse sequences having individual pulses with a spacing, resulting from the pulse train rate, with pulse durations between 0.05 und 50 µs,
- a detector for determining any bubble formation at the irradiation site while the first part-pulse sequence is emitted,
the control being switched for terminating the first pulse sequence and for starting the second pulse sequence, so that search for a therapeutically effective pulse energy no longer has to be carried out separately as soon as the detector applies a signal that is indicative of bubble formation in the tissue.

2. Laser according to Claim 1, **characterized in that** the control for outputting a second part-pulse sequence is designed with a plurality of pulses whose laser power is slightly below the end value of the first part-pulse sequence.

3. Laser according to Claim 1, **characterized by** a controllable dampening unit, provided outside the resonator, for the emitted laser radiation for subjecting it to a closed-loop control.

4. Laser according to Claim 3, **characterized by** means for pulse-shaping control of pre-determined laser pulses that are provided on a Q-switched laser.

5. Laser according to Claims 3 or 4, **characterized by** phase-change-detecting means of the detector for detecting the bubble formation that accompanies a phase change.

6. Laser according to Claims 3 to 5, **characterized by** interferometric means of the detector for detecting the bubble formation that detect the change in optical path lengths taking place during bubble formation.

7. Laser according to Claims 3 to 5, **characterized by** a photodiode of the detector for detecting the bubble formation, that detects the change in the refractive index taking place during bubble formation.

8. Laser for irradiating biological tissue during surgery with continuous online monitoring of the radiation effect on the tissues during the surgery, having:
- a control for emitting a single laser pulse having a pulse duration of up to 50 µs, whose pulse energy increases monotonously during a first time span while it is being emitted and remains essentially constant during a second time span while it is being emitted,
- a detector for determining any bubble formation in the biological tissue at the irradiation site during the first time span of emitting the laser pulse,
- the control being switched for terminating the first time span and for starting the second time span as soon as the detector applies a signal indicating bubble formation in the tissue, and for terminating the laser pulse after a given impact time that starts as soon as bubble formation is first established in the tissue, so that search for a therapeutically effective pulse energy no longer has to be carried out separately.

## Revendications

1. Laser pour l'irradiation de tissu biologique en intervention, avec observation en ligne en continu de l'effet de l'irradiation sur les tissus pendant l'intervention avec:
- une commande pour l'émission de séries d'impulsions laser de durée finie sous forme de salves, comprenant à chaque fois:
au moins deux séries d'impulsions partielles, d'au moins une impulsion chacune, appliquées à la suite,
avec une première série d'impulsions partielles présentant une puissance laser montant régulièrement en partant d'un niveau d'énergie d'impulsion faible prédéfini, et
avec une deuxième série d'impulsions partielles présentant une puissance laser en grande partie constante dans la plage de la valeur finale de la première série d'impulsions,
les impulsions individuelles des séries d'impulsions laser, composées de deux séries d'impulsions partielles, présentant un écart basé sur la vitesse d'enchaînement des séries d'impulsions avec des durées d'impulsions situées entre 0,05 et 50 µs
- un détecteur pour constater la formation éventuelle de cloques sur le site de l'irradiation pendant l'émission de la première série d'impulsions partielles,
la commande étant activée pour terminer la première série d'impulsions et débuter la deuxième série d'impulsions afin d'éviter de rechercher séparément une énergie d'impulsion à efficacité thérapeutique dès que le détecteur émet un signal indiquant la formation de cloques dans le tissu.

2. Laser selon revendication 1, **caractérisé par le fait que** la commande d'émission d'une deuxième série d'impulsions partielles dispense plusieurs impulsions dont la puissance laser se situe faiblement en dessous de la valeur finale de la première série d'impulsions partielles.

3. Laser selon revendication 1, **caractérisé par** une unité pilotable pour la réduction du rayonnement laser émis, prévue à l'extérieur du résonateur, pour assujettir le rayonnement à une régulation.

4. Laser selon revendication 3, **caractérisé par** des moyens de contrôle de la forme d'impulsions laser prédéfinies, prévus sur un laser à modulation de qualité.

5. Laser selon revendication 3 ou 4, **caractérisé par** des moyens de détection des changements de phases du détecteur de cloques procédant d'un changement de phase.

6. Laser selon les revendications 3 à 5, **caractérisé par** les moyens interférométriques du détecteur de cloques qui détectent la variation des trajets optiques lors de la formation de cloques.

7. Laser selon les revendications 3 à 5, **caractérisé par** une photodiode du détecteur de cloques qui détecte la variation de l'indice de réfraction lors de la formation de cloques.

8. Laser pour l'irradiation de tissu biologique en intervention, avec observation en ligne en continu de l'effet de l'irradiation sur les tissus pendant l'intervention avec:
- une commande d'émission d'une seule impulsion laser d'une durée maximale de 50 µs, dont la puissance monte régulièrement pendant la première phase de son émission et qui reste en grande partie constante pendant la deuxième phase de son émission,
- un détecteur pour détecter la formation éventuelle de cloques dans le tissu biologique sur le site de l'irradiation pendant la première phase d'émission de l'impulsion laser,
- la commande pour terminer la première phase et pour débuter la deuxième phase étant activée dès que le détecteur émet un signal indiquant la formation de cloques dans le tissu, et pour terminer l'impulsion laser après une durée d'action prescrite qui court dès la première constatation de la formation de cloques dans le tissu afin d'éviter de rechercher séparément une énergie d'impulsion à efficacité thérapeutique.
